# EUROPEAN PATENT SPECIFICATION

(11) **EP 2 060 294 B1**
(45) Date of publication and mention of the grant of the patent: **31.07.2013**
(21) Application number: 08169050.5
(22) Date of filing: 13.11.2008
(51) Int. Cl.: A61M 16/06

(54) **Headgear for use in non-invasive ventilation**
Kopfhalterung zur Verwendung bei nichtinvasiver Beatmung
Casque pour une utilisation dans une ventilation non invasive

(30) Priority: 13.11.2007 GB 0722249
(43) Date of publication of application: 20.05.2009
(73) Proprietor: Intersurgical AG, Vaduz (LI)
(72) Inventor: Bowsher, Richard Francis, Reading, Berkshire RG31 7DB (GB)
(74) Representative: Adamson Jones

(56) References cited:
- EP-A- 1 514 581
- WO-A-01/66194
- WO-A-2004/028637
- WO-A-2007/022562
- US-A- 5 441 046
- US-B1- 7 296 575

## Description

This invention relates to headgear for use in non-invasive ventilation.

Non-invasive ventilation is a process by which a flow of respiratory gas is delivered to the airway of a patient through a non-invasive interface device, such as a mask, and hence without using an endotracheal or tracheostomy tube. Non-invasive ventilation is typically used to manage both chronic and acute respiratory failure, and also other medical disorders, such as sleep apnea.

Presently available non-invasive interface devices include full face masks, nasal masks, nasal pillows, and mouthpieces. These interface devices are typically held in position by headgear. This is particularly important for masks, which generally have a sealing component that is urged against the face of the patient by the headgear.

Leakage from the interface device during use is undesirable because it reduces alveolar ventilation and synchrony between the patient and the ventilator, and hence patient comfort and/or the quality of sleep during use may be compromised. In order to minimise leakage from the interface device, it is important to ensure that there is a good fit between the interface device and the face of the patient, and also that the interface device is effectively urged against the patient's face. The action of the headgear is therefore very important for minimising leakage, whilst also minimising patient discomfort and irritation.

Conventional headgear typically comprises one or more straps that extend about the user's head, with means for adjusting the size of each strap in order to urge the interface device against the face of the patient with sufficient pressure in order to minimise leakage from the interface device. However, conventional types of headgear suffer several disadvantages. In particular, the straps of the headgear can become tangled and twisted during storage, and hence conventional headgear is often cumbersome to fit. Furthermore, it is often difficult to fit the headgear so that a substantially equal pressure is applied about the periphery of the interface device. Many conventional types of headgear also use hook-and-loop type fastenings, which can become entangled with a user's hair and may be difficult to grasp.

Document WO01/66194 discloses a disposable breathing mask attachable to the user's head by means of a strap in the form of a continuous loop of elasticated material, attachment books and guide means.

There has now been devised an improved headgear which overcomes or substantially mitigates the above-mentioned and/or other disadvantages associated with the prior art.

According to a first aspect of the invention, there is provided headgear for fastening a respiratory mask to the head of a user for use in non-invasive ventilation, the headgear comprising means for attaching the headgear to the respiratory mask, and first and second support members adapted to extend about the head of the user, in use, wherein the first and second support members are defined by a continuous member that is gripped at two separate points by at least one adjustment component to also define an adjustment loop, and adjustment of the size of the adjustment loop enables the sizes of the first and second support members to be varied.

The headgear according to the invention is advantageous principally in that adjustment of the size of the adjustment loop enables the sizes of both the first and second support members to be adjusted, thereby facilitating fitting of the headgear and the respiratory mask to the head of a user. In particular, the sizes of the first and second support members may be reduced simultaneously, until those support members are drawn against the head of the user, thereby enabling the respiratory mask to be readily fitted to the user. The headgear according to the invention is therefore less cumbersome to fit and also requires less adjustment actions for proper placement of the respiratory mask than prior art headgear.

The continuous member defines the first and second support members, as well as the adjustment loop. The continuous member may be defined by only one component, such as a single strap, but may also be defined by a plurality of components that are fixed together.

By "headgear" is meant a device that is adapted to fasten a respiratory mask device to the head of a user in an operative position, ie a position in which the respiratory mask is able to deliver gas to the respiratory system of the user. The headgear according to the invention preferably forms, together with the respiratory mask, non-invasive ventilation apparatus adapted to be fitted about the head of the user. Hence, according to a further aspect of the invention, there is provided non-invasive ventilation apparatus for delivering gas to the respiratory system of a user, the apparatus comprising a respiratory mask adapted for connection to a supply of respiratory gas, and headgear as described above, wherein the apparatus is adapted to be fitted about the head of the user, such that the respiratory mask is held in an operative position.

The headgear may be permanently attached to the respiratory mask, but is preferably removably attached to the respiratory mask so as to enable the headgear to be reused with other devices.. The respiratory mask may be a face mask, or a nasal mask, for example. Most preferably, the headgear is adapted for use with a mask comprising a rigid body that defines a cavity from which the user is able to inhale respiratory gas supplied to the mask, and a more flexible sealing component that seals the cavity of the mask to the face of the user. In this case, the headgear is preferably adapted to be attached to the rigid body of the mask.

The first and second support members preferably extend between two connection components, each connection component being adapted to be attached to the interface device. At least one of the connection components is preferably an adjustment component with which the continuous member cooperates so as to define an adjustment loop. The adjustment component is preferably situated approximately over the cheek area of the user, when the headgear is fitted, in order to be readily accessible to the user. In presently preferred embodiments, both of the connection components are adjustment components, such that the continuous member defines two adjustment loops, one on each side of the respiratory mask.

The means for attaching the headgear to the respiratory mask preferably comprises at least one engagement member that extends between a connection component and a mounting on the respiratory mask. In presently preferred embodiments, the engagement member extends between the connection components of the headgear, and is attached to the mounting of the respiratory mask in the region of its midpoint. In this case, two or more engagement members are preferably provided, so as to enable a substantially even pressure to be applied to the respiratory mask.

At least one of the connection components is preferably releasably attached to the respiratory mask, such that the headgear and respiratory mask may be readily removed from the head of the user. In particular, the apparatus preferably includes a quick-release mechanism, such that a single action of the user is sufficient to release the connection component from the respiratory mask, and hence disengage the apparatus from the head of the user. This quick-release mechanism may include a pull-cord, or the like. However, in presently preferred embodiments, the apparatus includes a clip that is adapted to engage a corresponding opening with a snap-fit, and includes a release member that enables the clip to be released from the opening. Most preferably, the release member is adapted to be depressed, towards the head of the user, in order to release the clip from the opening. This is typically achieved by means of a resiliently deformable engagement member that is fixed to a support member at one end, and includes a release member at the other end that enables the user to deform the engagement member.

The continuous member, and hence the first and second support members, is preferably formed of a flexible strip of material, such that the first and second support members have the form of straps. The first and second support members preferably extend over different portions of the head of the user, such that an upper support member extends about the crown area of the user's head, and a lower support member extends about the upper end of the user's neck. In this way, the first and second support members are preferably adapted, without additional support members, to fasten the respiratory mask to the head of the user. Where the support members have the form of straps, the headgear is preferably arranged so that a major surface of each member is in continual contact with the head of the user, and hence the support members are not twisted when fitted.

In order to maintain the position of the support members during use, the headgear preferably includes a strap support that extends between the first and second support members at a rear portion of the user's head, when fitted. Indeed, the continuous member, and hence the first and second support members, may be partly defined by the strap support. This strap support preferably has the form of a panel, which overlies a part of the rear of the user's head when fitted. In addition, the strap support is preferably adjustable in size, such that the distance between the first and second support members may be adjusted. For instance, the strap support may be foldable, and include one or more fasteners, such as hook-and-loop fasteners (eg Velcro® fasteners), for maintaining the strap support in its folded configuration.

The adjustment component is adapted to grip the continuous member at two separate points, such that the adjustment loop is defined between those points, and the support members extend from each end of the adjustment loop: The adjustment component preferably therefore includes two separate means for gripping the continuous member, which conveniently take the form of buckles through which the continuous member is threaded. Each buckle preferably defines an adjustment arm of the adjustment component, and extends in the direction of the rear of the user's head, when fitted, from a common support member. The buckles are preferably orientated at an angle to each other to reflect the different paths taken by the support members about the user's head.

For instance, the buckles may be orientated at an angle of between 30° and 70°, or more preferably between 40° and 60°, relative to each other. Each buckle preferably includes a pair of openings, the openings being separated by a crossmember. The continuous member is preferably adapted to pass through a first opening from the side of the buckle nearest the user's head, about the crossmember, and hence through the second opening, and back over the portion of the continuous member that leads to the first opening, such that the continuous member doubles-back on itself in the direction of the rear of the user's head, when fitted. Hence, for each adjustment component, the associated adjustment loop of the continuous member preferably extends from those portions of the continuous member that are clamped between an adjacent portion of the continuous member and a surface of the adjustment component.

The flexible material of the continuous member is preferably substantially inextensible in form, and preferably comprises webbing. The strap support preferably includes a cushion layer for improving the comfort of the user during use, which may be formed of a compressible cellular material. However, in presently preferred embodiments, the strap support has a greater rigidity than the first and second support members, such that it maintains its shape during fitting of the headgear to a greater extent than the continuous member, thereby facilitating fitting of the headgear to the head of the user. This greater rigidity may be achieved, for example, by having a cushion layer with a substantially increased thickness relative to that of the continuous member. In presently preferred embodiments, the cushion layer of the strap support is interposed between two layers of fabric material. The strap support may also include reinforcing layers or members in order to provide greater rigidity.

The strap support is preferably provided with an opening, which may have the form of a slot, through which parts of the support members may be threaded. In this way, the connection components may be drawn alongside the strap support, such that the headgear adopts a collapsed configuration. The support members and/or the strap support may include pre-formed folds that facilitate collapse of the headgear in this manner. Most preferably, the strap support of the headgear retains the support straps in a manner in which the risk of the support straps becoming twisted and/or tangled is reduced. According to a further aspect of the invention, there is provided a method of fitting non-invasive ventilation apparatus to the head of a user, the non-invasive apparatus comprising the headgear described above and a respiratory mask, the method comprising arranging the first and second support members about the head of the user, arranging the respiratory mask in an operative position on the face of the user, and increasing the size of the adjustment loop until the first and second support members are drawn against the head of the user with a desired force.

In presently preferred embodiments, one or more parts of the adjustment loop are urged away from the adjustment component, such that the continuous member is simultaneously drawn through the adjustment component, thereby simultaneously reducing the size of the first and second support members, until the support members are drawn against the user's head with a desired force. In order to perform this action, a user may grip portions of the adjustment loop adjacent to each gripping means of the adjustment component, typically using two hands, and urge those portions away from the adjustment component in an appropriate direction. Alternatively, a user may grip an intermediate region of the adjustment loop, typically with one hand, but with several fingers, and urge that intermediate region away from the adjustment component. If either the first support member or the second support member is drawn against the user's head with a desired force before the other support member, continued urging of an appropriate part of the adjustment loop away from the adjustment component will cause the untightened support member to reduce in size until it is also drawn against the user's head with a desired force. In this way, both the first and second support members of the headgear may be drawn against the user's head.

Where the headgear includes two adjustment loops, and hence two adjustment components, the user may either increase the size of both adjustment loops simultaneously, or alternatively increase the size of the adjustment loops in alternate increments, until the first and second support members are drawn against the user's head to a sufficient degree. Furthermore, rather than simultaneously adjust the size of the first and second support members, it is also possible for a user to grasp a part of the adjustment loop that is adjacent to an adjustment arm, and draw the continuous member through that adjustment arm, in order to adjust the size of one support member independently of the other support member.

Furthermore, each gripping means of the adjustment component is preferably releasable, so that the adjustment loop may be reduced in size, thereby increasing the size of the first and second support members. In particular, where the gripping means are buckles, the user preferably urges an end portion of a buckle away from the face of the user, so as to enable the continuous strap to be released from the grip of the buckle. The headgear may therefore be loosened during use, for increased comfort, or may be loosened sufficiently to be removed from the head of the user following use.

A preferred embodiment of the invention will now be described in greater detail, by way of illustration only, with reference to the accompanying drawings, in which
Figure 1 is first perspective view of non-invasive ventilation apparatus according to the invention, including headgear and a mask;
Figure 2 is a second perspective view of the non-invasive ventilation apparatus;
Figure 3 is a front view of the non-invasive ventilation apparatus;
Figure 4 is fragmentary view of the headgear, which illustrates tightening of the headgear;
Figure 5 is a fragmentary view of the headgear, which is partially cut-away and illustrates loosening of the headgear;
Figure 6 is a fragmentary view of the headgear, illustrating removal of the head gear; and
Figure 7 is a third perspective view of the non-invasive ventilation apparatus, which illustrates collapse of the headgear into a storage configuration.

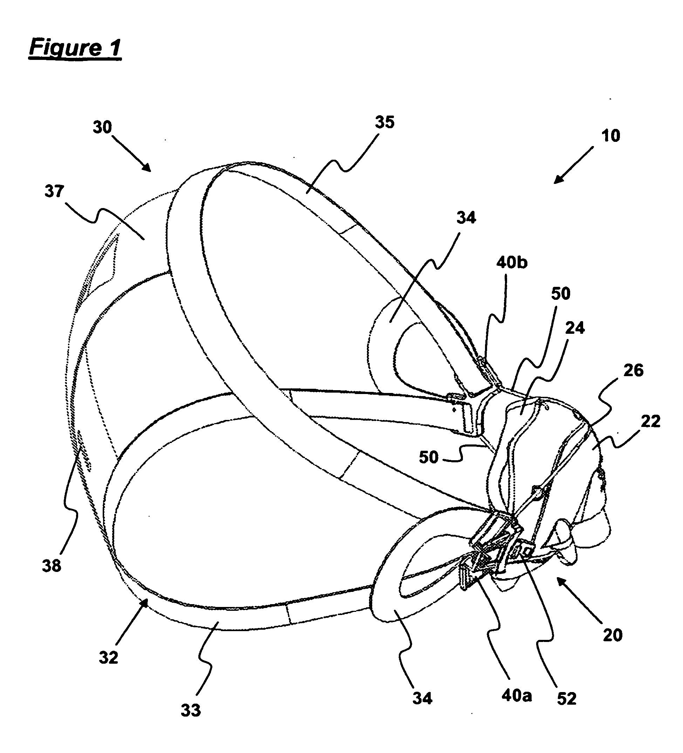

Figures 1 to 3 show non-invasive ventilation apparatus according to the invention, which is generally designated 10. The non-invasive ventilation apparatus 10 comprises a face mask 20 and headgear 30. The face mask 20 comprises a relatively rigid body 22 adapted to be supplied with respiratory gas, and a sealing component 24 detachably fastened to the periphery of the mask body 22 and adapted to contact the face of a patient during use. The sealing component 24 is adapted to form an effective seal between the face mask 20 and the face of the user to which the apparatus 10 is fitted. The detachable fastening between the mask body 22 and the sealing component 24 is achieved by means of integrally formed mating formations of the mask body 22 and the sealing component 24.

The headgear 30 comprises a continuous strap 32 that cooperates with a pair of adjustment components 40a,40b in order to define a lower support strap 33, an upper support strap 35, and a pair of adjustment loops 34. The headgear 30 is adapted to be fitted to a user so that the upper and lower support straps 33,35 extend between the adjustment components 40a,40b, around the rear of the user's head, and the adjustment loops 34 are disposed on each side of the user's head. The upper and lower support straps 33,35 are also arranged so that a major surface of each of those support straps 33,35 is in continuous contact with the user's head, and the support straps 33,35 are not twisted when fitted.

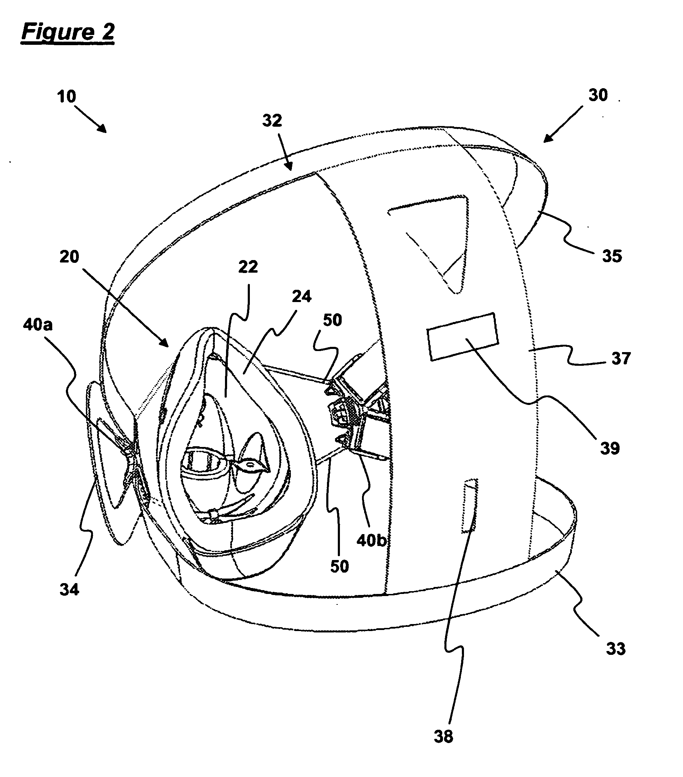

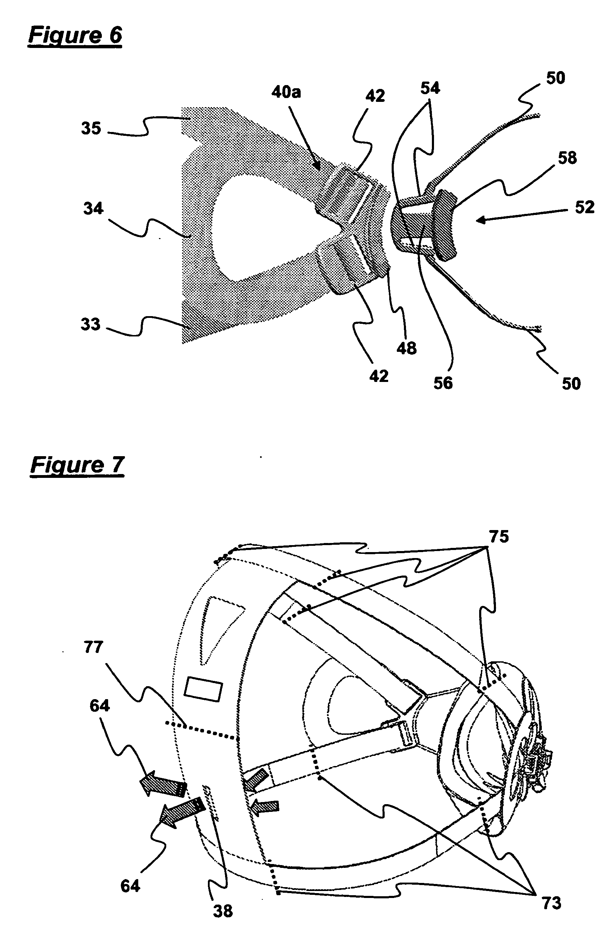

The headgear 30 also includes a support panel 37 that extends between fixings at a mid-portion of the upper strap 35 and a mid-potion of the lower strap 33, such that the support panel 37 overlies a rear portion of a user's head when fitted. The support panel 37 includes a narrow, rectangular opening 38, with which the continuous strap 32 is engaged during collapse of the headgear 30, as discussed in more detail below in relation to Figure 7. In addition, as shown most clearly in Figure 2, the rear surface of the support panel 37 includes a fastener 39 comprising an area of hook-and-loop material (eg. a Velcro® fastener) that is adapted to fasten to an adjacent area of the rear surface of the support panel 37. This arrangement enables the support panel 37 to be folded, thereby reducing its length and hence the distance between the upper and lower support straps 33,35, and maintained in its folded configuration by the fastener 39. This feature therefore enables the headgear 30 to be used with a greater range of sizes of head.

The continuous strap 32 and the support panel 37 are both formed of a flexible material, and are fixed together by stitching. During manufacture, the strip of flexible material of the continuous strap 32 is firstly engaged with the adjustment components 40a,40b, as described in more detail below, before being stitched together at its ends. The flexible material of the continuous strap 32 comprises a substantially inextensible webbing material. The flexible material of the support panel 37 comprises two layers of fabric, between which is disposed a cushion layer formed of a compressible cellular material. However, the support panel 37 has a greater rigidity than the continuous strap 32, such that the support panel 37 is able to maintain its shape during fitting of the headgear 10 to a greater extent than the continuous strap 32, thereby facilitating fitting of the headgear 30 to the head of the user. In addition, the cushion layer of the support panel 37 reduces any discomfort experienced by the user during use.

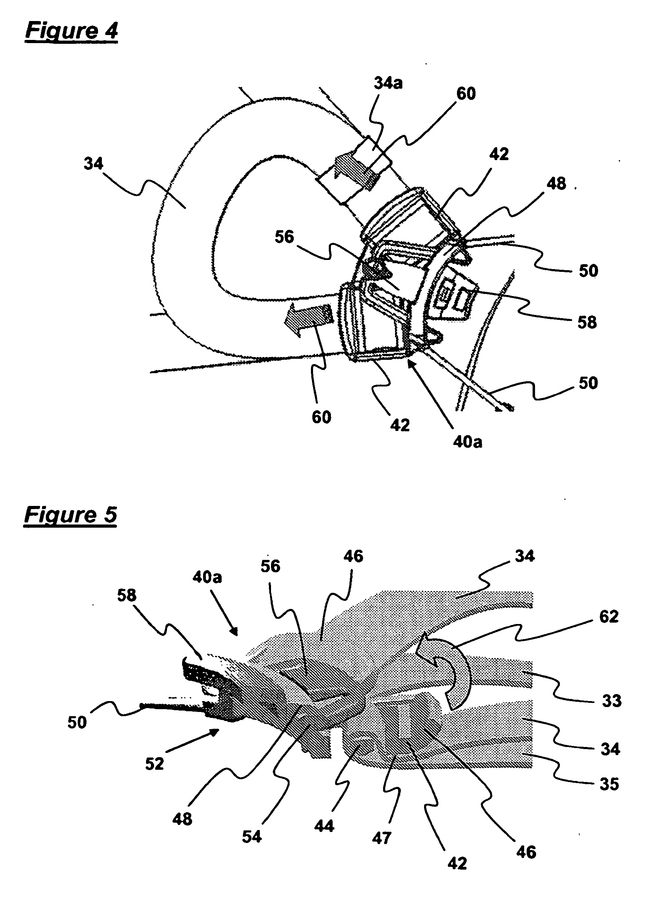

A first adjustment component 40a of the headgear 30 is shown most clearly in Figures 4 to 6. The adjustment component 40a comprises a pair of adjustment arms 42 that extend from adjacent halves of an arcuate support member 48. The adjustment arms 42 are identical in form, and are orientated at an angle of approximately 50° to each other. Each adjustment arm 42 is generally rectangular in shape, with a front opening and a rear opening separated by cross-member 44, as shown most clearly in Figure 5. The engaged continuous strap 32 is doubled-back on itself, about the cross-member 44 of the adjustment arm 42, such that the doubled continuous strap 32 extends over a surface of the adjustment arm 42 that faces the user when fitted. This arrangement causes the adjustment arm 42 to grip the continuous strap 32, when fitted. In order to improve the grip between the adjustment arm 42 and the continuous strap 32, the adjustment arm 42 includes a lug 47 with a relatively narrow projecting edge formed on the surface over which the continuous strap 32 extends.

As shown in Figures 1 to 3, both the first and second adjustment components 40a,40b include adjustment arms 42 and an arcuate support member 44, as described above. The continuous strap 32 is engaged with the adjustment arms 42 of the first and second adjustment components 40a,40b, so as to define the upper and lower support straps 33,35 and the pair of adjustment loops 34. In particular, the part of the continuous strap 32 that defines the lower support strap 33 passes through the front and rear openings of a lower adjustment arm 42 of the first adjustment component 40a, and then defines the adjustment loop 34 for that adjustment component 40a, before passing through the front and rear openings of the upper adjustment arm 42 of the first adjustment component 40a, and then extending to the second adjustment component 40b to define the upper support strap 35. Similarly, the part of the continuous strap 32 that defines the upper support strap 35 passes through the front and rear openings of the upper adjustment arm 42 of the second adjustment component 40b, and then defines the adjustment loop 34 for that adjustment component 40b, before passing through the front and rear openings of the lower adjustment arm 42 of the second adjustment component 40b, and then extending to the first adjustment component 40a to define the lower support strap 33.

For each adjustment component 40a,40b, the associated adjustment loop 34 of the continuous strap 32 extends from those portions of the continuous strap 32 that are clamped between an adjacent portion of the continuous strap 32 and a surface of the adjustment arm 42, as shown most clearly in Figure 5.

If the parts of the adjustment loop 34 adjacent to the adjustment arms 42 are urged away from the adjustment component 40a,40b, as indicated by arrows 60 in Figure 4, the continuous strap 32 will be drawn through both of the adjustment arms 42, so that the adjustment loop 34 is enlarged. This action will therefore simultaneously reduce the size of the upper and lower support straps 33,35, until the upper and lower support straps 33,35 are drawn against the user's head with a desired force. In order to perform this action, a user may grip portions of the adjustment loop 34 adjacent to each adjustment arm 42, typically using two hands, and urge those portions away from the adjustment component 40a,40b in the general direction in which the associated adjustment arm 42 is orientated. Alternatively, a user may grip an intermediate region of the adjustment loop 34, typically with one hand, but with several fingers, and urge that intermediate region away from the adjustment component 40a,40b.

If either the upper support strap 35 or the lower support strap 33 is drawn against the user's head with a desired force before the other support strap 33,35, continued urging of the portion of the adjustment loop 34 associated with the untightened support strap 33,35 away from the adjustment component 40a,40b will cause the untightened support strap 33,35 to reduce in size until it is also drawn against the user's head with a desired force. In this way, both the upper and lower support straps 33,35 of the headgear 30 may be drawn against the user's head. Where a user is urging an intermediate region of the adjustment loop 34 away from the adjustment component 40a,40b, this may be achieved by exerting a force on the adjustment loop 34 using only one end of the user's grip, for instance by rotating the hand.

In order to facilitate this process, a user may either increase the size of both adjustment loops 34 simultaneously, or alternatively increase the size of the adjustment loops 34 in alternate increments, until the upper and lower support straps 33,35 are drawn against the user's head to a sufficient degree. Furthermore, rather than simultaneously adjust the size of the support straps 33,35, it is also possible for a user to grasp a part of the adjustment loop 44 that is adjacent to an adjustment arm 42, and draw the continuous strap 32 through that adjustment arm 42, in order to adjust the size of one support strap 33,35 independently of the other support strap 33,35.

In addition, an end portion 46 of each adjustment arm 42 is inclined relative to the remainder of the adjustment arm 42, such that it extends away from the user's face, when fitted. This configuration facilitates a user urging the end portion 46 of the adjustment arm 42 away from the face of the user, so as to enable the continuous strap 32 to be released from the grip of the attachment arm 42. This action is illustrated by arrow 62 in Figure 5, and enables the size of the associated adjustment loop 34 to be reduced, and hence the size of the first support strap 33 and/or second support strap 35 to be increased, by enabling the continuous strap 32 to be fed through the openings of one, or both, of the adjustment arms 42. The headgear 30 may therefore be loosened during use, for increased comfort, or may be loosened sufficiently to be removed from the head of the user following use.

In order to retain the adjustment loops 34 against the side of the user's head, without impeding adjustment of the size of the adjustment loops 34, as discussed above, the headgear 30 also includes a retaining loop 34a for each adjustment loop 34. The retaining loop 34a for one of the adjustment loops 34 is illustrated in Figure 4, but is not shown in the remaining Figures. The retaining loop 34 is a loop of material that extends about either the upper or lower support strap 33,35 (in this case the upper support strap 35) as well as an adjacent part of the associated adjustment loop 34, and hence retains the adjustment loop 34 adjacent to the upper and lower support straps 33,35 whilst enabling relative movement therebetween.

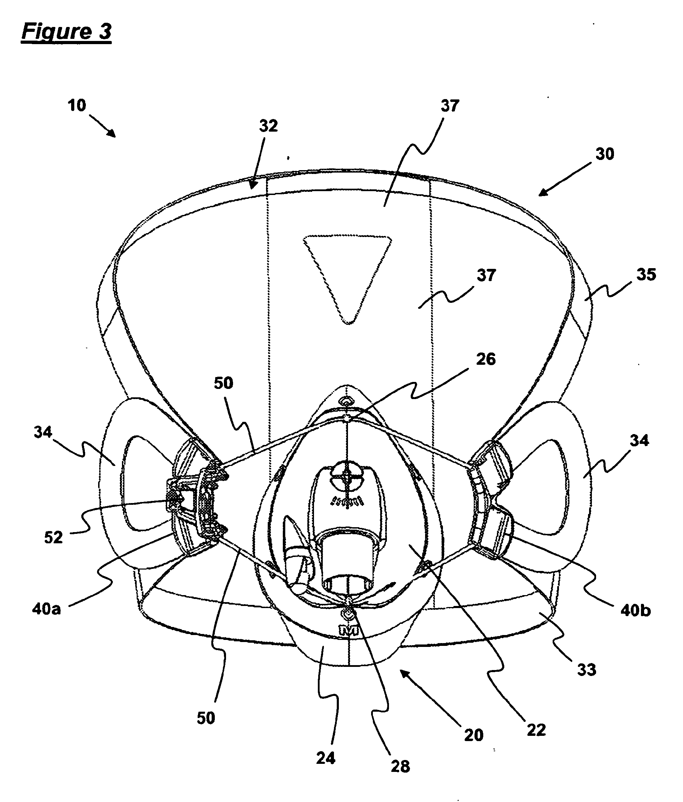

Both of the adjustment components 40a,40b of the headgear 30 include the adjustment arms 42 and arcuate support member 48 described above, as shown in Figure 3. However, the first adjustment component 40a also includes an opening in the arcuate support member 44 with which a clip 52 is engaged. Furthermore, a connection thread 50 extends from each side of the clip 52 to each end of the arcuate support member 48 of the second adjustment member 40b. The engagement thread 50 and clip 52 together connect the headgear 30 to the face mask 20. In particular, the connection thread 50 extends from the clip 52 at one end of the arcuate support member 48 of the first adjustment component 40a, through a groove 26 formed transversely across a bridge portion of the mask body 22, to a corresponding end of the arcuate support member 48 of the second adjustment member 40b. The other connection thread 50 extends from the other end of the arcuate support member 48 of the second adjustment member 40b, back though a groove 28 formed transversely across a chin portion of the mask body 22, and terminates at the clip 52 at the corresponding end of the arcuate support member 48 of the first adjustment component 40a. Each groove 26,28 of the face mask 20 includes a retaining member that prevents the engagement thread 50 disengaging from the grooves 26,28 during use.

As shown clearly in Figure 6, the clip 52 comprises a pair of side arms 54 having curved ends that meet at a front end of the clip 52, and a central engagement part that extends rearward from the front end of the clip 52, between the pair of side arms 54. The pair of side arms 54 are adapted to be closely received within each end of the opening in the arcuate support member 48 of the first adjustment component 40a. The central engagement part of the clip 52 includes a front engagement member 56 and a rear release member 58. The front engagement member 56 and the rear release member 58 define a recess therebetween, and are both raised out of the plane of the side arms 54. The rear release member 58 is adapted to be depressed by a user in order to release the clip 52 from the adjustment component 42, in use.

The central engagement part of the clip 52 is adapted such that when the front end of the clip 52 is inserted into the opening in the support member 48 of the first adjustment component 40a, the raised nature of the front engagement member 56 causes the central engagement part of the clip 52 to be deformed away from its original position relative to the side arms 54, towards the plane of the side arms 54. When the front engagement member 56 has passed through the opening, the central engagement part of the clip 52 returns to its original position relative to the side arms 54, such that the support member 48 is located within the recess of the central engagement part of the clip 52, between the front engagement member 56 and the rear release member 58, and hence the clip 52 is fastened to the adjustment component 40a.

In order to release the clip 52 from the adjustment component 40a, the user depresses the rear release member 58, towards the face of the user, such that the front engagement member 56 is able to pass through the opening in the support member 48, and the clip 52 is removed from that opening. This enables the headgear 30 to be rapidly disengaged from the user's head, such that the headgear 30 is readily removable by the user.

The headgear 30 is supplied in a collapsed configuration in thermo-formed packaging (not shown in the Figures). The collapsed configuration is arranged so that the headgear 30 is readily expandable into a configuration suitable for fitting to a user's head. In particular, Figure 7 illustrates the approximate position of pre-formed folds 73,75,77 in the headgear 30, along which the headgear 30 is folded in its collapsed configuration. In particular, the upper and lower support straps 33,35 each include a pre-formed fold 73,75 on each side of the support panel 37, and a further pre-formed fold 73,75 towards each end of the support straps 33,35. In addition, the support panel 37 includes a pre-formed fold 77 that extends transversely across the support panel 37.

Furthermore, the four parts of the continuous strap 32 that extend between an adjustment component 40a,40b and the support panel 37 are adapted to be folded about a fold 73,75, and threaded through the opening 38 in the support panel 37, such that the adjustment components 40a,40b are brought alongside the support panel 37. This collapsed configuration ensures that the continuous strap 32 does not become twisted and/or tangled during storage or normal handling, and a user may readily extend the adjustment components 40a,40b from the strap support 37, before fitting the headgear 30.

## Claims

1. Headgear (30) for fastening a respiratory mask (20) to the head of a user for use in non-invasive ventilation, the headgear (30) comprising means for attaching the headgear (30) to the respiratory mask (20), and first and second support members (33,35) adapted to extend about the head of the user, in use, wherein the first and second support members (33,35) are defined by a continuous member (32), **characterised in that** the continuous member (32) is gripped at two separate points by at least one adjustment component (40a,40b) to define an adjustment loop (34), and adjustment of the size of the adjustment loop (34) enables the sizes of the first and second support members (33,35) to be varied.

2. Headgear (30) as claimed in Claim 1, wherein the first and second support members (33,35) extend between two connection components (40a,40b), each connection component (40a,40b) being adapted to be attached to the respiratory mask (20).

3. Headgear (30) as claimed in Claim 2, wherein at least one of the connection components (40a,40b) is an adjustment component (40a,40b) with which the continuous member (32) cooperates so as to define an adjustment loop (34).

4. Headgear (30) as claimed in Claim 2 or Claim 3, wherein at least one of the connection components (40a,40b) is releasably attached to the respiratory mask (20), such that the headgear (30) and respiratory mask (20) may be readily removed from the head of the user.

5. Headgear (30) as claimed in Claim 4, wherein the apparatus includes a quick-release mechanism, such that a single action of the user is sufficient to release the connection component (40a,40b) from the respiratory mask (20), and hence disengage the apparatus from the head of the user.

6. Headgear (30) as claimed in any preceding claim, wherein the first and second support members (33,35) are adapted, without additional support members (33,35), to fasten the respiratory mask (20) to the head of the user.

7. Headgear (30) as claimed in any preceding claim, wherein the first and second support members (33,35) are adapted to extend over different portions of the head of the user, such that an upper support member (35) extends about the crown area of the user's head, and a lower support member (33) extends about the upper end of the user's neck.

8. Headgear (30) as claimed in any preceding claim, wherein the adjustment component (40a,40b) is adapted to grip the continuous member (32) at two separate points, such that the adjustment loop (34) is defined between those points, and the support members (33,35) extend from each end of the adjustment loop (34).

9. Headgear (30) as claimed in any preceding claim, wherein the headgear (30) includes a strap support (37) that extends between the first and second support members (33,35) at a rear portion of the user's head, when fitted.

10. Headgear (30) as claimed in Claim 9, wherein the strap support (37) is adjustable in size, such that the distance between the first and second support members (33,35) may be adjusted.

11. Headgear (30) as claimed in Claim 10, wherein the strap support (37) is foldable, and includes one or more fasteners for maintaining the strap support in its folded configuration.

12. Non-invasive ventilation apparatus for delivering gas to the respiratory system of a user, the apparatus comprising a respiratory mask (20) adapted for connection to a supply of respiratory gas, and headgear (30) as claimed in any preceding claim, wherein the apparatus is adapted to be fitted about the head of the user, such that the respiratory mask (20) is held in an operative position.

13. Non-invasive ventilation apparatus as claimed in Claim 12, wherein the headgear (30) is removably attachable to the respiratory mask (20).

14. A method of fitting non-invasive ventilation apparatus to the head of a user, the non-invasive apparatus comprising the headgear (30) as claimed in any one of Claims 1 to 11 and a respiratory mask (20), the method comprising arranging the first and second support members (33,35) about the head of the user, arranging the respiratory mask (20) in an operative position on the face of the user, and increasing the size of the adjustment loop (34) until the first and second support members (33,35) are drawn against the head of the user with a desired force.

15. A method as claimed in Claim 14, wherein one or more parts of the adjustment loop (34) are urged away from the adjustment component (40a,40b), such that the sizes of the first and second support members (33,35) are simultaneously reduced, until the support members (33,35) are drawn against the user's head with a desired force.

## Patentansprüche

1. Kopfgeschirr (30) zum Befestigen einer Beatmungsmaske (20) am Kopf eines Benutzers für eine nicht-invasive Beatmung, wobei das Kopfgeschirr (30) Mittel zum Anbringen des Kopfgeschirrs (30) an der Beatmungsmaske (20) sowie ein erstes und zweites Stützelement (33, 35) umfasst, die so gestaltet sind, dass sie beim Gebrauch um den Kopf des Benutzers verlaufen, wobei das erste und zweite Stützelement (33, 35) durch ein kontinuierliches Element (32) definiert werden, **dadurch gekennzeichnet, dass** das kontinuierliche Element (32) an zwei separaten Punkten von wenigstens einer Justierkomponente (40a, 40b) erfasst wird, um eine Justierschlaufe (34) zu definieren, und durch Justieren der Größe der Justierschlaufe (34) die Größe des ersten und zweiten Stützelements (33, 35) variiert werden kann.

2. Kopfgeschirr (30) nach Anspruch 1, wobei das erste und zweite Stützelement (33, 35) zwischen zwei Verbindungskomponenten (40a, 40b) verlaufen, wobei jede Verbindungskomponente (40a, 40b) zum Anbringen an der Beatmungsmaske (20) gestaltet ist.

3. Kopfgeschirr (30) nach Anspruch 2, wobei wenigstens eine der Verbindungskomponenten (40a, 40b) eine Justierkomponente (40a, 40b) ist, mit der das kontinuierliche Element (32) zusammenwirkt, um eine Justierschlaufe (34) zu definieren.

4. Kopfgeschirr (30) nach Anspruch 2 oder Anspruch 3, wobei wenigstens eine der Verbindungskomponenten (40a, 40b) lösbar an der Beatmungsmaske (20) angebracht ist, so dass das Kopfgeschirr (30) und die Beatmungsmaske (20) leicht vom Kopf des Benutzers abgenommen werden können.

5. Kopfgeschirr (30) nach Anspruch 4, wobei die Vorrichtung einen Schnelllösemechanismus aufweist, so dass mit einer einzigen Tätigkeit des Benutzers die Verbindungskomponente (40a, 40b) von der Beatmungsmaske (20) gelöst und die Vorrichtung vom Kopf des Benutzers abgenommen werden kann.

6. Kopfgeschirr (30) nach einem der vorherigen Ansprüche, wobei das erste und zweite Stützelement (33, 35) zum Befestigen, ohne zusätzliche Stützelemente (33, 35), der Beatmungsmaske (20) am Kopf des Benutzers ausgelegt sind.

7. Kopfgeschirr (30) nach einem der vorherigen Ansprüche, wobei das erste und zweite Stützelement (33, 35) so gestaltet sind, dass sie über verschiedene Abschnitte des Kopfes des Benutzers verlaufen, so dass ein oberes Stützelement (35) um den Scheitelbereich des Kopfes des Benutzers verläuft und ein unteres Stützelement (33) um das obere Ende des Halses des Benutzers verläuft.

8. Kopfgeschirr (30) nach einem der vorherigen Ansprüche, wobei die Justierkomponente (40a, 40b) zum Erfassen des kontinuierlichen Elementes (32) an zwei separaten Punkten ausgelegt ist, so dass die Justierschlaufe (34) zwischen diesen Punkten definiert wird, und die Stützelemente (33, 35) sich von jedem Ende der Justierschlaufe (34) erstrecken.

9. Kopfgeschirr (30) nach einem der vorherigen Ansprüche, wobei das Kopfgeschirr (300) eine Riemenhalterung (37) aufweist, die, wenn sie angebracht ist, zwischen dem ersten und zweiten Stützelement (33, 35) an einem hinteren Abschnitt des Kopfes eines Benutzers verläuft.

10. Kopfgeschirr (30) nach Anspruch 9, wobei die Gräße der Riemenhalterung (37) so justiert werden kann, dass der Abstand zwischen dem ersten und dem zweiten Stützelement (33, 35) justiert werden kann.

11. Kopfgeschirr (30) nach Anspruch 10, wobei die Riemenhalterung (37) faltbar ist und ein oder mehrere Befestigungselemente zum Halten der Riemenhalterung in ihrer gefalteten Konfiguration aufweist.

12. Nicht-invasive Beatmungsvorrichtung zum Zuführen von Gas zum Beatmungssystem eines Benutzers, wobei die Vorrichtung eine Beatmungsmaske (20) zum Verbinden mit einer Beatmungsgasversorgung sowie ein Kopfgeschirr (30) nach einem der vorherigen Ansprüche umfasst, wobei die Vorrichtung so ausgelegt ist, dass sie so um den Kopf des Benutzers gesetzt werden kann, dass die Beatmungsmaske (20) in einer Betriebsposition gehalten wird.

13. Nicht-invasive Beatmungsvorrichtung nach Anspruch 12, wobei das Kopfgeschirr (30) entfernbar an der Beatmungsmaske (20) angebracht werden kann.

14. Verfahren zum Montieren einer nicht-invasiven Beatmungsvorrichtung am Kopf eines Benutzers, wobei die nicht-invasive Vorrichtung das Kopfgeschirr (30) nach einem der Ansprüche 1 bis 11 und eine Beatmungsmaske (20) umfasst, wobei das Verfahren das Anordnen des ersten und zweiten Stützelements (33, 35) um den Kopf des Benutzers, das Anordnen der Beatmungsmaske (20) in einer Betriebsposition auf dem Gesicht des Benutzers und das Vergrößern der Justierschlaufe (34) umfasst, bis das erste und zweite Stützelement (33, 35) mit einer gewünschten Kraft an den Kopf des Benutzers gezogen werden.

15. Verfahren nach Anspruch 14, wobei ein oder mehrere Teile der Justierschlaufe (34) von der Justierkomponente (40a, 40b) weg gedrückt wird/werden, so dass die Größen des ersten und zweiten Stützelements (33, 35) gleichzeitig reduziert werden, bis die Stützelemente (33, 35) mit einer gewünschten Kraft an den Kopf des Benutzers gezogen werden.

## Revendications

1. Casque (30) servant à attacher un masque respiratoire (20) à la tête d'un utilisateur pour un usage de ventilation non invasive, le casque (30) comprenant un moyen pour fixer le casque (30) au masque respiratoire (20), et des premier et deuxième organes de support (33, 35) conçus pour s'étendre autour de la tête de l'utilisateur, quand il est utilisé, les premier et deuxième organes de support (33, 35) étant définis par un organe continu (32) **caractérisé en ce que** l'organe continu (32) est saisi en deux points séparés par au moins un composant d'ajustement (40a, 40b) pour définir une boucle d'ajustement (34), et qu'un ajustement de la dimension de la boucle d'ajustement (34) permet de varier les dimensions des premier et deuxième organes de support (33, 35).

2. Casque (30) selon la revendication 1, dans lequel les premier et deuxième organes de support (33, 35) s'étendent entre deux composants de connexion (40a, 40b), chaque composant de connexion (40a, 40b) étant conçus pour être fixé au masque respiratoire (20).

3. Casque (30) selon la revendication 2, dans lequel au moins un des composants de connexion (40a, 40b) est un composant d'ajustement (40a, 40b) avec lequel l'organe continu (32) coopère de manière à définir une boucle d'ajustement (34).

4. Casque (30) selon la revendication 2 ou la revendication 3, dans lequel au moins un des composants de connexion (40a, 40b) est fixé de manière amovible au masque respiratoire (20), pour que le casque (30) et le masque respiratoire (20) puissent être faciles à retirer de la tête de l'utilisateur.

5. Casque (30) selon la revendication 4, dans lequel l'appareil comprend un mécanisme à détachement rapide, faisant qu'une seule action de l'utilisateur soit suffisante pour détacher le composant de connexion (40a, 40b) du masque respiratoire (20), et séparer ainsi l'appareil de la tête de l'utilisateur.

6. Casque (30) selon l'une quelconque des revendications précédentes, dans lequel les premier et deuxième organes de support (33, 35) sont conçus, sans organe de support supplémentaire (33, 35), pour attacher le masque respiratoire (20) à la tête de l'utilisateur.

7. Casque (30) selon l'une quelconque des revendications précédentes, dans lequel les premier et deuxième organes de support (33, 35) sont conçus pour s'étendre sur différentes parties de la tête de l'utilisateur, de manière qu'un organe de support supérieur (35) s'étende autour du haut du crâne de l'utilisateur, et qu'un organe de support inférieur (33) s'étende autour de l'extrémité supérieur du col de l'utilisateur.

8. Casque (30) selon l'une quelconque des revendications précédentes, dans lequel le composant d'ajustement (40a, 40b) est conçu pour saisir l'organe continu (32) en deux points séparés, pour que la boucle d'ajustement (34) soit définie entre ces points, et que les organes de support (33, 35) s'étendent à partir de chaque extrémité de la boucle d'ajustement (34).

9. Casque (30) selon l'une quelconque des revendications précédentes, dans lequel le casque (30) comprend un support de sangle (37) qui s'étend entre les premier et deuxième organes de support (33, 35) à une partie arrière de la tête de l'utilisateur, quand il est installé.

10. Casque (30) selon la revendication 9, dans lequel le support de sangle (37) a une taille réglable, pour que la distance entre les premier et deuxième organes de support (33, 35) puisse être ajustée.

11. Casque (30) selon la revendication 10, dans lequel le support de sangle (37) est pliable et comprend une ou plusieurs attaches pour maintenir le support de sangle à sa configuration pliée.

12. Appareil de ventilation non invasive servant à fournir du gaz au système respiratoire d'un utilisateur, l'appareil comprenant un masque respiratoire (20) conçu pour être connecté à une alimentation de gaz respiratoire, et un casque (30) selon l'une quelconque des revendications précédentes, l'appareil étant conçu pour être installé autour de la tête de l'utilisateur, pour que le masque respiratoire (20) soit maintenu à une position opérationnelle.

13. Appareil de ventilation non invasive selon la revendication 12, dans lequel le casque (30) peut être fixé de manière amovible au masque respiratoire (20).

14. Procédé d'installation d'appareil de ventilation non invasive à la tête d'un utilisateur, l'appareil non invasif comprenant le casque (30) selon l'une quelconque des revendications 1 à 11 et un masque respiratoire (20), le procédé consistant à agencer les premier et deuxième organes de support (33, 35) autour de la tête de l'utilisateur, agencer le masque respiratoire (20) à une position opérationnelle sur le visage de l'utilisateur, et à augmenter la taille de la boucle d'ajustement (34) jusqu'à ce que les premier et deuxième organes de support (33, 35) soient attirés contre la tête de l'utilisateur avec une force souhaitée.

15. Procédé selon la revendication 14, dans lequel une ou plusieurs parties de la boucle d'ajustement (34) sont éloignées du composant d'ajustement (40a, 40b), pour que les dimensions des premier et deuxième organes de support (33, 35) soient simultanément réduites, jusqu'à ce que les organes de support (33, 35) soient attirés contre la tête de l'utilisateur avec une force souhaitée.
